# EUROPEAN PATENT APPLICATION

(11) **EP 3 270 418 A1**
(43) Date of publication of application: **17.01.2018**
(21) Application number: 15884679.0
(22) Date of filing: 09.11.2015
(51) Int. Cl.: H01L 27/14, A61B 1/04, G02B 23/24, H01L 21/3205, H01L 21/768, H01L 21/822, H01L 23/522, H01L 27/04, H01L 27/146, H04N 5/369

(54) **SIZE REDUCTION OF IMAGING DEVICE**

(30) Priority: 11.03.2015 JP 2015048711
(71) Applicant: OLYMPUS CORPORATION, Hachioji-shi Tokyo 192-8507 (JP); Panasonic Corporation, Kadoma-shi, Osaka 571-8501 (JP)
(72) Inventor: YAMASHITA, Tomokazu, Tokyo 192-8507 (JP); IGARASHI, Takatoshi, Tokyo 192-8507 (JP); FUJIMORI, Noriyuki, Tokyo 192-8507 (JP); KATSUNO, Motonari, Osaka 540-6207 (JP)
(74) Representative: Gunzelmann, Rainer
(86) International application number: PCT/JP2015/081480
(87) International publication number: WO 2016/143195

(57) **Abstract**

Provided is an imaging device which is highly reliable and can be miniaturized by preventing peeling of the insulating member. An imaging unit 40 of the present invention has a semiconductor chip 44 having an image sensor formed thereon, and a protective glass 49 adhered on the image sensor with an adhesive layer 54c. The semiconductor chip 44 includes a light-receiving section 44a which photoelectrically converting the input light to generate an image signal; a peripheral circuit section 44b which receives the image signal from the light-receiving section 44a and transmits a driving signal; a guard ring 44d which surrounds the light-receiving section 44a and the peripheral circuit section 44b; and a plurality of metal dots 44e formed on an outer circumference of the guard ring 44d. The protective glass 49 is adhered by the adhesive layer 54c to cover the light-receiving section 44a, the peripheral circuit section 44b, the guard ring 44d, and the metal dots 44e.

## Description

### Field

The present invention relates to miniaturization of an imaging device which is provided at a distal end of an insertion section of an endoscope to be inserted into a subject to capture an image of an interior of the subject. Background

Conventionally, endoscope devices have been widely used for various inspections in a medical field and an industrial field. Among them, since a medical endoscope device is capable of obtaining an in-vivo image even without incision of the subject, by inserting a flexible insertion section having an elongated shape in which an imaging device is provided at the distal end into a subject such as a patient, and is capable of performing a therapeutic treatment by causing a treatment tool to protrude from the distal end of the insertion section as necessary, the medical endoscope device is widely used.

The imaging device used in such an endoscope device includes a semiconductor chip on which an image sensor is formed, and a circuit board on which electronic components such as capacitors or IC chips constituting a drive circuit of the image sensor are mounted, and a signal cable is soldered to the circuit board. The semiconductor chip has a peripheral circuit section which transmits and receives signals between a light-receiving section and external components, on a semiconductor substrate having the light-receiving section formed thereon. In recent years, however, in order to improve the performance of the imaging device, Low-k film of low dielectric constant is used as the material of the insulating layer of the semiconductor chip.

Since the Low-k film is inferior in moisture resistance, if the Low-k film is exposed to the outer circumferential portion of the semiconductor chip, water penetrates into the insulating layer, which may cause a malfunction or corrosion of the metal wiring. Thus, there has been proposed an imaging device in which a guard ring made of a material having excellent moisture resistance is formed on the outer circumference of the light-receiving sections and the like in a plurality of insulating members of the semiconductor chip having the light-receiving sections formed thereon (see, for example, Patent Literature 1).

### Citation List

### Patent Literature

Patent Literature 1: JP 2014-216554 A

### Summary

### Technical Problem

In the imaging device of Patent Literature 1, although a protective glass which protects the light-receiving section or the like of the semiconductor chip is adhered to the semiconductor chip by an adhesive layer, the Low-k film is exposed on the outer circumference of the adhesive surface of the semiconductor chip. Since the Low-k film is inferior in adhesion to the adhesive layer or a sealing resin and is mechanically fragile, there is a risk of occurrence of peeling between the adhesive surface and the insulating layer. Further, since there is a difference in linear expansion between the protective glass and the semiconductor chip, when thermal stress is applied, there is a high risk of occurrence of peeling of the Low-k film. Further, since an objective optical system is disposed by a frame member on an opposite side of a connecting surface of the protective glass to the semiconductor chip, when an external force is applied to the objective optical system, stress is concentrated in the Low-k film which is a joining end portion between the protective glass and the semiconductor chip, the possibility of peeling increases, and the reliability of the imaging device is significantly lowered.

The present invention has been made in view of the above, and an object thereof is to provide an imaging device which is highly reliable and can be miniaturized by preventing peeling of an insulating member such as a Low-k film.

### Solution to Problem

To solve the above-described problem and achieve the object, an imaging unit according to the present invention includes: a semiconductor chip including an image sensor formed thereon; and a protective glass adhered on the image sensor with an adhesive layer, wherein the semiconductor chip includes: a light-receiving section configured to generate an image signal by performing photoelectric conversion of light; a peripheral circuit section configured to receive the image signal from the light-receiving unit and transmit a driving signal to the light-receiving unit; a guard ring surrounding the light-receiving section and the peripheral circuit section; and a plurality of metal dots formed on an outer circumference of the guard ring, and the protective glass is adhered by the adhesive layer so as to cover the light-receiving section, the peripheral circuit section, the guard ring, and the metal dots.

Moreover, in the above-described imaging unit according to the present invention, the adhesive layer includes a hollow portion located above the light-receiving section, and the protective glass is adhered by the adhesive layer so as to cover the peripheral circuit section, the guard ring, and the metal dots.

Moreover, in the above-described imaging unit according to the present invention, the metal dots are further formed on a portion which is not covered with the protective glass, on a surface of the semiconductor chip on which the light-receiving section is formed, and a sealing resin is filled on the metal dots formed in the portion not covered with the protective glass, and is adhered to a side surface of the protective glass.

Moreover, in the above-described imaging unit according to the present invention, a sealing resin is filled in a portion which is not in contact with the semiconductor chip, on a connecting surface between the protective glass and the semiconductor chip, and is adhered to a side surface of the semiconductor chip.

Moreover, in the above-described imaging unit according to the present invention, the guard ring and the metal dots are formed of dummy vias and dummy pads formed on a plurality of insulating members laminated on a semiconductor substrate on which the light-receiving section is formed, respectively, and the plurality of insulating members are Low-k films.

Moreover, an endoscope device according to the present invention includes an insertion section including any one of the above-described imaging units provided at a distal end.

### Advantageous Effects of Invention

Since a plurality of metal dots is provided on the outer circumferential portion of the connecting surface between the semiconductor chip and the protective glass, even when stress is applied to the adhesive surface between the semiconductor chip and the protective glass, by the miniaturization of the imaging device of the present invention, it is possible to prevent peeling of the insulating member such as the laminated Low-k film.

### Brief Description of Drawings

FIG. 1 is a diagram schematically illustrating an overall configuration of an endoscope system according to a first embodiment of the present invention.
FIG. 2 is a partial cross-sectional view of a distal end of the endoscope device illustrated in FIG. 1.
FIG. 3 is a plan view of a semiconductor chip used in the imaging unit of FIG. 2.
FIG. 4 is a partial cross-sectional view of the imaging unit of FIG. 2.
FIG. 5 is an enlarged cross-sectional view of a metal dot of FIG. 4.
FIG. 6 is a partially enlarged view illustrating a modified example of the metal dot.
FIG. 7 is a partial cross-sectional view of an imaging unit according to a modified example of the first embodiment of the present invention.
FIG. 8 is a partial cross-sectional view of an imaging unit according to a second embodiment of the present invention.
FIG. 9 is a plan view of a semiconductor chip used in the imaging unit of FIG. 8.
FIG. 10 is a partial cross-sectional view of an imaging unit according to a modified example of the second embodiment of the present invention.
FIG. 11 is a partial cross-sectional view of an imaging unit according to a third embodiment of the present invention.
FIG. 12A is a partial cross-sectional view of an imaging unit according to a fourth embodiment of the present invention.
FIG. 12B is a front view of the imaging unit according to the fourth embodiment of the present invention.
FIG. 13 is a plan view of a semiconductor chip used in an imaging unit according to a fifth embodiment of the present invention.
FIG. 14 is a partial cross-sectional view of an imaging unit according to the fifth embodiment of the present invention.

### Description of Embodiments

In the following description, an endoscope device provided with an imaging unit will be described as modes for carrying out the present invention (hereinafter referred to as "embodiments"). Further, the present invention is not limited by such embodiments. Furthermore, in the description of the drawings, the same parts are denoted by the same reference numerals. Furthermore, the drawings are schematic, a relation between the thickness and the width of each member, a ratio of each member and the like are different from the reality. In addition, portions having dimensions and ratios different from each other are also included in the drawings.

### (First Embodiment)

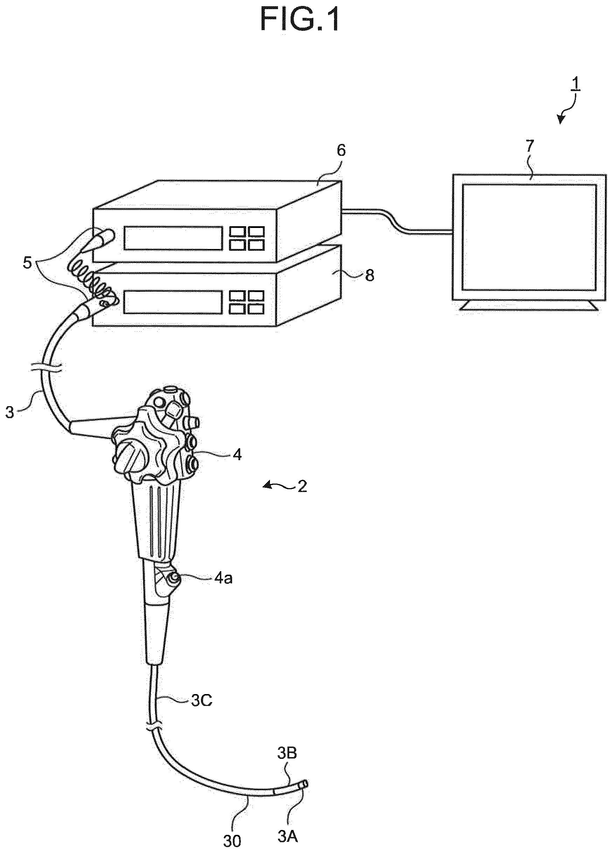

FIG. 1 is a diagram schematically illustrating an overall configuration of an endoscope system according to an embodiment of the present invention. As illustrated in FIG. 1, an endoscope system 1 includes an endoscope device 2, a universal cord 3, a connector unit 5, a processor (control device) 6, a display device 7, and a light source device 8.

The endoscope device 2 captures an in-vivo image of a subject and outputs an image signal, by inserting an insertion section 30 into the subject. An electric cable bundle inside the universal cord 3 extends to the insertion section 30 of the endoscope device 2, and is connected to the imaging unit provided at a distal end portion 3A of the insertion section 30.

An operating unit 4 provided with various buttons and knobs which operate the endoscope function is connected to a proximal end side of the insertion section 30 of the endoscope device 2. The operating unit 4 is provided with a treatment tool insertion port 4a through which treatment tools such as a biological forceps, an electric scalpel and a test probe are inserted into the body cavity of the subject.

The connector unit 5 is provided at the proximal end of the universal cord 3, and is connected to the light source device 8 and the processor 6 to perform predetermined signal processing on the image signal which is output from the imaging device of the distal end portion 3A connected to the universal cord 3 and to perform an analog-to-digital conversion (A/D conversion) of the image signal and output the image signal.

The processor 6 performs predetermined image processing on the image signal which is output from the connector unit 5, and controls the entire endoscope system 1. The display device 7 displays the image signal processed by the processor 6.

The pulsed white light turned on by the light source device 8 is illumination light that is emitted from the distal end of the insertion section 30 of the endoscope device 2 toward the subject via the universal cord 3 and the connector unit 5. The light source device 8 is configured, for example, using a white LED.

The insertion section 30 includes a distal end portion 3A on which the imaging device is provided, a bending portion 3B connected to the proximal end side of the distal end portion 3A and freely bendable in a plurality of directions, and a flexible tube section 3C connected to the proximal end side of the bending portion 3B. The image signal of the image captured by the imaging device provided at the distal end portion 3A is connected, for example, to the connector unit 5 via the operating unit 4 by the universal cord 3 having the length of several meters. The bending portion 3B is bent by operating a bending operation knob provided on the operating unit 4, and is freely bendable in four directions, for example, upward, downward, rightward, and leftward along with the pulling and loosening of the bending wire inserted into the insertion section 30.

A light guide bundle (not illustrated) which transmits the illumination light from the light source device 8 is disposed in the endoscope device 2, and an illumination lens (not illustrated) is disposed at an emission end of the illumination light by the light guide bundle. The illumination lens is provided at the distal end portion 3A of the insertion section 30, and the illumination light is emitted toward the subject.

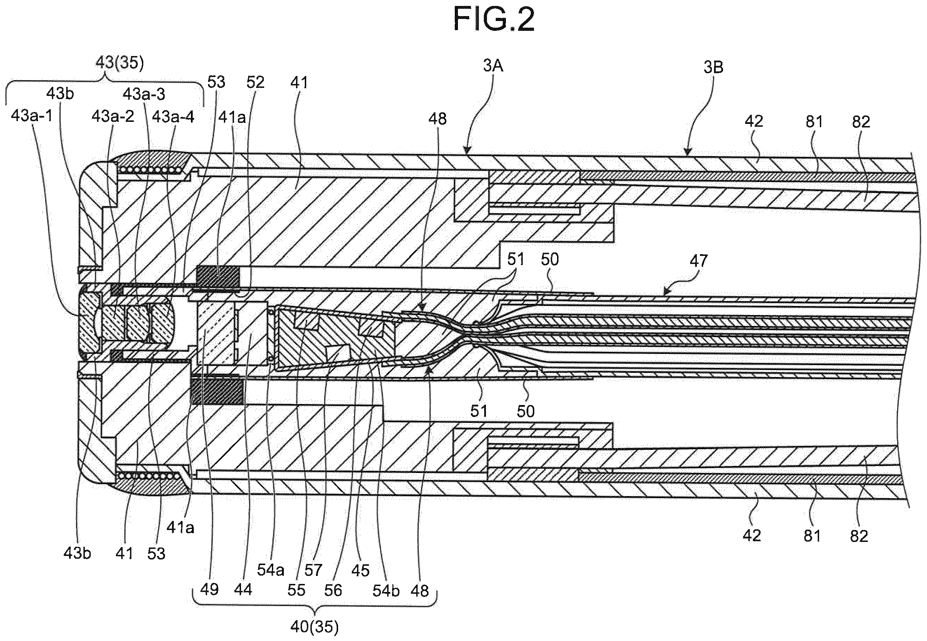

Next, the configuration of the distal end portion 3A of the endoscope device 2 will be described in detail. FIG. 2 is a partial cross-sectional view of the distal end of the endoscope device 2. In FIG. 2, a distal end portion 3A of the insertion section 30 of the endoscope device 2 and a part of the bending portion 3B are illustrated.

As illustrated in FIG. 2, the bending portion 3B is freely bendable in four directions, upward, downward, leftward, and rightward together with pulling and loosening of a bending wire 82 inserted into a bending tube 81 disposed inside a cladding tube 42 to be described later. An imaging device 35 is provided inside the distal end portion 3A extending to the distal end side of the bending portion 3B.

The imaging device 35 has a lens unit 43, and an imaging unit 40 disposed on the proximal end side of the lens unit 43, and is adhered to the interior of a distal end portion main body 41 with an adhesive 41a. The distal end portion main body 41 is formed of a hard member for forming an internal space which stores the imaging device 35. The proximal end outer circumferential portion of the distal end portion main body 41 is covered with a flexible cladding tube 42. The member closer to the proximal end side than the distal end portion main body 41 is made of a flexible member so that the bending portion 3B can be bent. The distal end portion 3A in which the distal end portion main body 41 is disposed serves as a hard portion of the insertion section 30.

The lens unit 43 has a plurality of objective lenses 43a-1 to 43a-4, and a lens holder 43b which holds the objective lenses 43a-1 to 43a-4. When the distal end of the lens holder 43b is inserted and fixed to the interior of the distal end portion main body 41, the lens unit 43 is fixed to the distal end portion main body 41.

The imaging unit 40 includes a semiconductor chip 44 having a light-receiving section which generates an image signal by receiving light such as CCD or CMOS to perform the photoelectric conversion, a flexible printed circuit board 45 (hereinafter referred to as "FPC board 45") which is bent in a U shape and is connected to the back side of the light-receiving surface of the semiconductor chip 44 on a surface serving as a U-shaped bottom surface portion, and a protective glass 49 adhered to the semiconductor chip 44 in a state of covering the light-receiving surface of the semiconductor chip 44. On the FPC board 45, electronic components 55 to 57 constituting the drive circuit of the image sensor formed on the semiconductor chip 44 are mounted. The electronic components 55 to 57 are mounted inside the U-shaped bent portion of the FPC board 45, and the inner side of the FPC board 45 bent in a U shape and mounted with the electronic components 55 to 57 is sealed with a sealing resin 54b. Further, the distal ends of each signal cable 48 of an electric cable bundle 47 are connected to the proximal end side of the FPC board 45. Electronic components other than electronic components constituting the drive circuit of the image sensor may be mounted on the FPC board 45.

The proximal ends of each signal cable 48 extend in the proximal end direction of the insertion section 30. The electric cable bundle 47 is disposed to be inserted through the insertion section 30 and extends to the connector unit 5, via the operating unit 4 and the universal cord 3 illustrated in FIG. 1.

The subject image formed by the objective lenses 43a-1 to 43a-4 of the lens unit 43 is detected by the light-receiving section of the semiconductor chip 44 disposed at the image forming positions of the objective lenses 43a-1 to 43a-4, and is converted into an image signal. The image signal is output to the processor 6 via the signal cable 48 connected to the FPC board 45 and the connector unit 5.

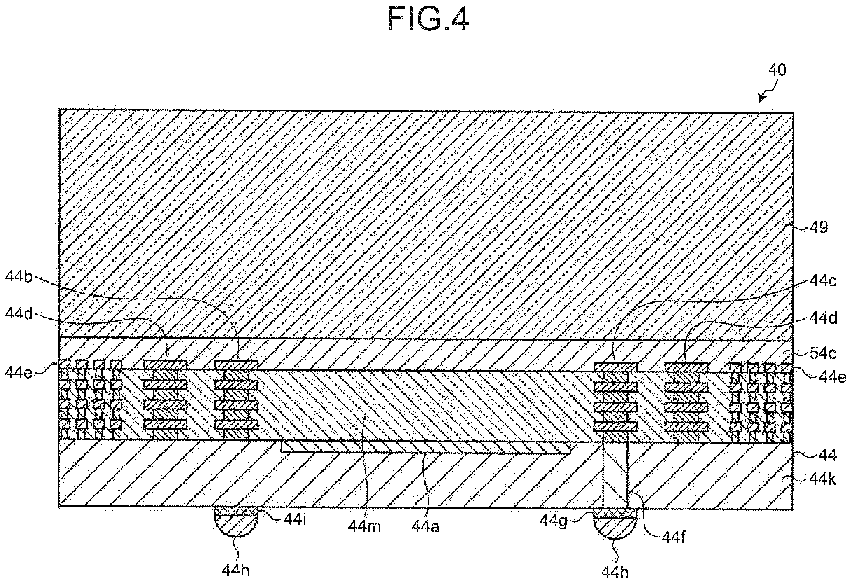

The semiconductor chip 44 is connected to the FPC board 45 by a bump 44h (see FIG. 4), and the connection circumference between the semiconductor chip 44 and the FPC board 45 is filled with a sealing resin 54a. The semiconductor chip 44, and the connecting section between semiconductor chip 44 and the FPC board 45 are covered with a metal reinforcing member 52. In order to prevent the influence of external static electricity on the electronic components 55 to 57 on the FPC board 45, the reinforcing member 52 is installed apart from the semiconductor chip 44 and the FPC board 45.

The outer circumference of the imaging unit 40 and the distal end portion of the electric cable bundle 47 is covered with a heat shrinkable tube 50 in order to improve resistance. Inside the heat shrinkable tube 50, a gap between the components is filled with an adhesive resin 51.

An image sensor holder 53 holds the semiconductor chip 44 adhered to the protective glass 49, by fitting the outer circumferential surface of the protective glass 49 to the inner circumferential surface on the proximal end side of the image sensor holder 53. The proximal end side outer circumferential surface of the image sensor holder 53 is fitted to the distal end side inner circumferential surface of the reinforcing member 52. A proximal end side outer circumferential surface of the lens holder 43b is fitted to the distal end side inner circumferential surface of the image sensor holder 53. In the state in which the respective members are fitted to each other, the outer circumferential surface of the lens holder 43b, the outer circumferential surface of the image sensor holder 53, and the distal end side outer circumferential surface of the heat shrinkable tube 50 are fixed to the inner circumferential surface of the distal end of the distal end portion main body 41 by the adhesive 41a.

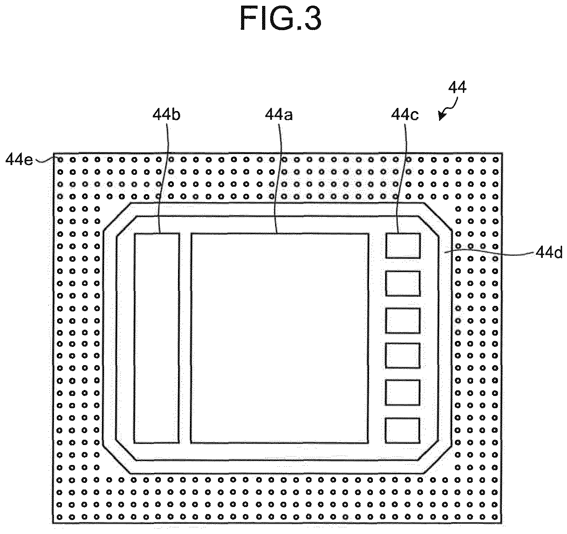

Next, the imaging unit 40 will be described. FIG. 3 is a plan view of the semiconductor chip 44 used in the imaging unit 40. FIG. 4 is a partial cross-sectional view of the imaging unit according to the first embodiment of the present invention, and illustrates a cross-sectional view of a connecting section between the protective glass 49 of the imaging unit 40 and the semiconductor chip 44.

The semiconductor chip 44 includes a light-receiving section 44a which performs photoelectric conversion of the light input from the lens unit 43 to generate an image signal, a peripheral circuit section 44b which receives the image signal from the light-receiving section 44a and transmits the driving signal to the light-receiving section 44a, a plurality of electrode pads 44c, a guard ring 44d which surrounds the light-receiving section 44a, the peripheral circuit section 44b and the electrode pad 44c, and a plurality of metal dots 44e formed on the outer circumference of the guard ring 44d. The protective glass 49 is formed to have the same planar dimensions orthogonal to the optical axis direction as the semiconductor chip 44, and is adhered by an adhesive layer 54c to cover the light-receiving section 44a, the peripheral circuit section 44b, the electrode pad 44c, the guard ring 44d, and the plurality of metal dots 44e.

The light-receiving section 44a is formed on a semiconductor substrate 44k made of silicon or the like.
On a surface opposite to the surface on which the light-receiving section 44a of the semiconductor substrate 44k is formed, the same number of back electrodes 44g and dummy electrodes 44i as the electrode pads 44c are formed. The back electrode 44g is formed at the same position as the position at which the electrode pad 44c of the semiconductor substrate 44k is formed, and is made conductive by a through-electrode 44f. The dummy electrode 44i is formed to be symmetrical with the back electrode 44g, and maintains a constant connection interval between the semiconductor chip 44 and the FPC board 45 when connected to the FPC board 45 via the bump 44h.

On the surface of the semiconductor substrate 44k on which the light-receiving section 44a is formed, an insulating layer 44m made up of a plurality of insulating members is laminated. In the insulating layer 44m of the first embodiment, insulating members are laminated in four layers, but the number of layers on which the insulating member is laminated is not limited thereto. As the insulating member, it is preferable to use a material having a low dielectric constant, and for example, a Low-k film with SIO₂ or resin as a base material can be suitably used. Since the Low-k film has a low dielectric constant, speed of the signal transmission in the wiring layer can be enhanced.

The peripheral circuit section 44b and the electrode pad 44c are formed by electrically connecting a via disposed in each insulating member constituting the insulating layer 44m and the wiring layer disposed on the insulating member.

The guard ring 44d is provided to surround the light-receiving section 44a, the peripheral circuit section 44b, and the electrode pad 44c, and to traverse in the thickness direction of the insulating layer 44m from the surface side of the insulating layer 44m abutting on the semiconductor substrate 44k to the surface side abutting on the adhesive layer 54c. Thus, moisture is prevented from entering the inner region of the guard ring 44d. The guard ring 44d is made of a metal material such as copper used as a material of the peripheral circuit section 44b.

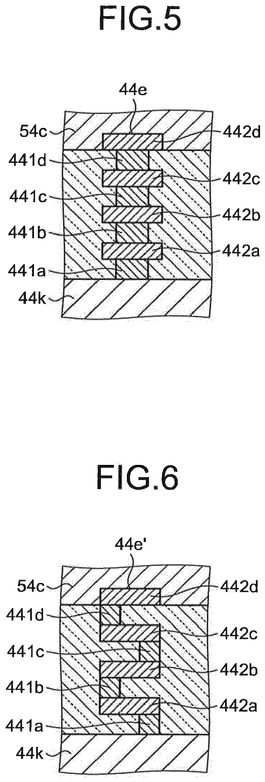

The metal dot 44e is made of a metal material such as copper, and a plurality of metal dots 44e is formed on the outer circumferential side of the guard ring 44d. In the first embodiment, four rows of metal dots 44e are formed in the up-down direction and the left-right direction on the outer circumference of the guard ring 44d. FIG. 5 illustrates an enlarged cross-sectional view of the metal dot 44e. The metal dot 44e includes a dummy via 441a formed in the first insulating member, a dummy pad 442a formed on the first insulating member, a dummy via 441b formed in the second insulating member, a dummy pad 442b formed on the second insulating member, a dummy via 441c formed in the third insulating member, a dummy pad 442c formed on the third insulating member, a dummy via 441d formed in the fourth insulating member, and a dummy pad 442d formed on the fourth insulating member. The diameters of the dummy pads 442a to 442d are approximately 5 µm, and the metal dots 44e are disposed at a pitch in which the dummy pads do not interfere with each other. The diameter of the dummy pad is not limited to this size. The metal dots 44e are disposed at the same interval, but the arrangement interval may be changed, for example, so that the inner side close to the guard ring 44d is dense and the outer side is sparse. The dummy vias 441a to 441d and the dummy pads 442a to 442d are disposed to abut on each other so as to be located at the same position in the thickness direction of the insulating layer 44m from the surface side of the insulating layer 44m abutting on the semiconductor substrate 44k to the surface side abutting on the adhesive layer 54c. As in the metal dots 44e, the guard ring 44d is also formed by disposing the dummy vias disposed in each insulating member and the dummy pads disposed on the insulating member constituting the insulating layer 44m to abut on each other.

In the first embodiment, even if a Low-k film or the like which is inferior in adhesion and is mechanically fragile is used as the insulating member of the semiconductor chip 44, since a plurality of metal dots 44e is disposed at the connection end portion of the connecting surface between the semiconductor chip 44 susceptible to stress and the protective glass 49, peeling of the insulating member can be prevented. After forming a large number of semiconductor chips 44 at a time, the semiconductor chip 44 is diced at a predetermined position to divide the semiconductor chips 44. However, by forming the metal dots 44e on the outer circumferential portion of the semiconductor chip 44, it is possible to prevent peeling of the insulating layer 44m at the time of dicing.

Further, the metal dots 44e may be disposed such that the dummy vias 441a to 441d may be disposed to be shifted in the thickness direction of the insulating layer 44m. FIG. 6 is a partially enlarged view illustrating a modified example of a metal dot. As illustrated in FIG. 6, in a metal dot 44e' according to the modified example, the dummy vias 441a to 441d are disposed to be shifted in zigzag in the thickness direction of the insulating layer 44m. Even when the dummy vias 441a to 441d are disposed to be shifted in the thickness direction of the insulating layer 44m, since the dummy vias 441a to 441d and the dummy pads 442a to 442d are disposed to abut on each other from the surface side of the insulating layer 44m abutting on the semiconductor substrate 44k to the surface side abutting on the adhesive layer 54c, it is possible to prevent peeling of the laminated insulating layers 44m, when stress is applied to the connection end portion between the semiconductor chip 44 and the protective glass 49.

Further, by forming the metal dots 44e in the diced portion in the wafer before dividing the semiconductor chip 44, it is possible to effectively prevent peeling of the insulating layer 44m or chipping of the semiconductor substrate 44k. When forming the metal dots 44e in the diced portion, if the dummy vias 441a to 441d are disposed to be shifted in the thickness direction of the insulating layer 44m as in the metal dots 44e' according to the modified example, the consumption of the dicing blade can be reduced.

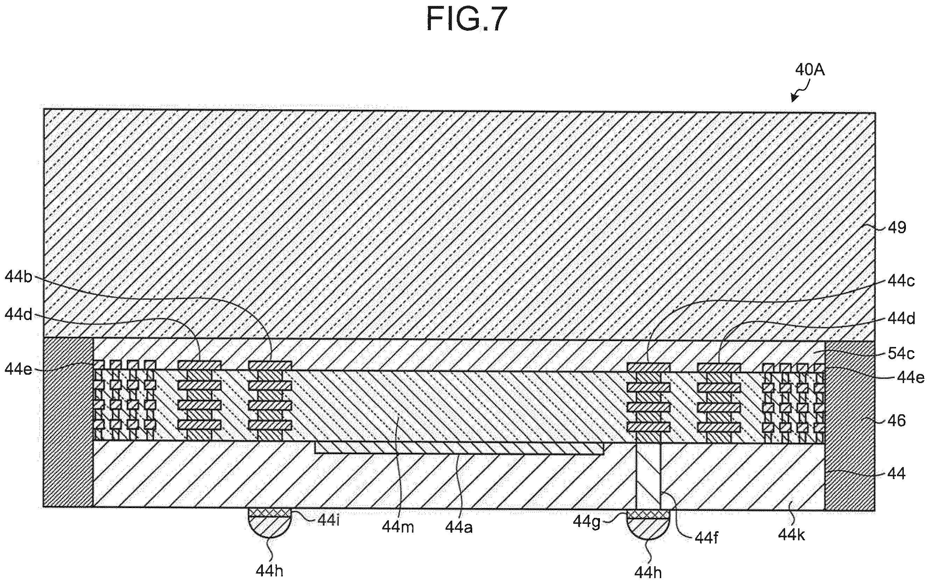

In addition, when the planar dimension of the protective glass 49 orthogonal to the optical axis direction is larger than that of the semiconductor chip 44, it is possible to fill the sealing resin and prevent peeling of the insulating member from the side surface direction of the semiconductor chip 44. FIG. 7 is a partial cross-sectional view of an imaging unit according to a modified example of the first embodiment of the present invention. FIG. 7 is a cross-sectional view of a connecting section between the protective glass 49 and the semiconductor chip 44 of the imaging unit according to the modified example of the first embodiment of the present invention.

In the imaging unit 40A according to the modified example of the first embodiment of the present invention, the protective glass 49 has a planar dimension orthogonal to the optical axis direction larger than that of the semiconductor chip 44. On the connecting surface of the protective glass 49 with the semiconductor chip 44, a portion which does not abut on the semiconductor chip 44 is filled with a sealing resin 46, and the side surface of the semiconductor chip 44 and the outer circumferential portion of the connecting surface of the protective glass 49 are adhered by a sealing resin 46. By sealing the side surface of the semiconductor chip 44 with the sealing resin 46, it is possible to prevent peeling of the insulating member from the side surface direction of the semiconductor chip 44.

### (Second Embodiment)

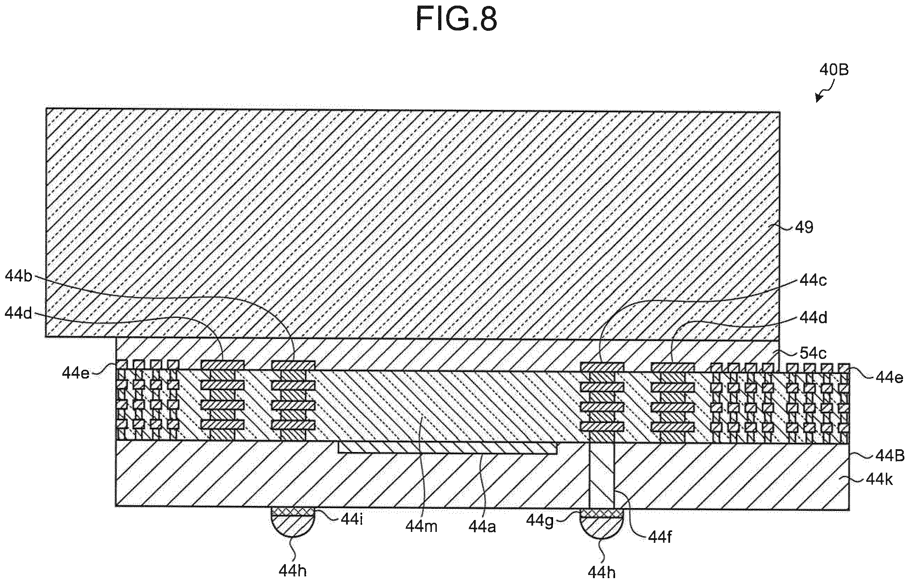

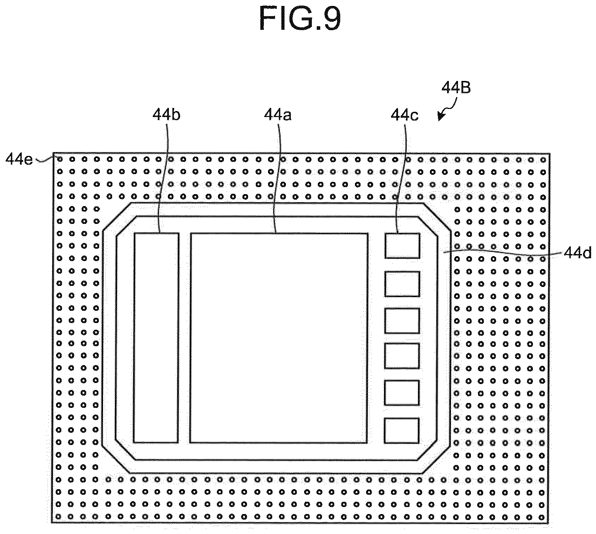

FIG. 8 is a partial cross-sectional view of an imaging unit according to a second embodiment of the present invention. FIG. 8 is a cross-sectional view of a connecting section between the protective glass 49 and a semiconductor chip 44B of the imaging unit according to the second embodiment of the present invention. FIG. 9 is a plan view of a semiconductor chip used in the imaging unit of FIG. 8.

In an imaging unit 40B according to the second embodiment, as illustrated in FIG. 9, four rows of metal dots 44e are formed on the upper and lower sides and the left side of the guard ring 44d, and eight rows of metal dots 44e are formed on the right side. Further, the left side of the guard ring 44d is the left side when viewed in the plan view of FIG. 9 (the outer circumference side of the guard ring 44d close to the peripheral circuit section 44b), and the right side is the right side when viewed in the plan view of FIG. 9 (the outer circumferential side of the guard ring 44d close to the electrode pad 44c).

The protective glass 49 is adhered by the adhesive layer 54c to cover the light-receiving section 44a, the peripheral circuit section 44b, the electrode pad 44c, the guard ring 44d, and the metal dots 44e of four rows of upper, lower, right and left sides. Among the metal dots 44e formed in eight rows on the right side of the guard ring 44d, the inner four rows of metal dots 44e are covered with the protective glass 49, but the outer four rows of metal dots 44e are not covered with a protective glass.

In the second embodiment, all the metal dots 44e are not covered with the protective glass 49. However, since a plurality of metal dots 44e is disposed at the connection end portion of the connecting surface between the semiconductor chip 44B prone to stress and the protective glass 49, even when a Low-k film or the like which is inferior in adhesion and mechanically fragile is used as an insulating member of the semiconductor chip 44B, peeling of the insulating member can be prevented.

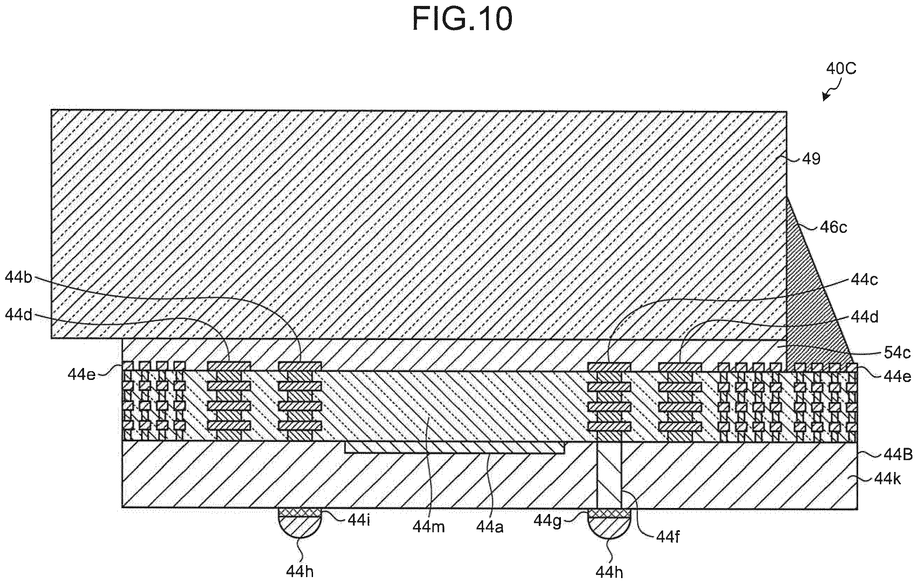

Further, sealing resin may be filled on the metal dots 44e of the semiconductor chip 44B not covered with the protective glass 49 to prevent peeling of the insulating member. FIG. 10 is a partial cross-sectional view of an imaging unit according to a modified example of the second embodiment of the present invention. FIG. 10 illustrates a cross-sectional view of a connecting section between the protective glass 49 and the semiconductor chip 44B of the imaging unit according to the modified example of the second embodiment of the present invention.

In an imaging unit 40C according to the modified example of the second embodiment of the present invention, a sealing resin 46c is filled on the metal dots 44e of the semiconductor chip 44B which is not covered with the protective glass 49, and the connecting surface of the semiconductor chip 44B and the side surface of the protective glass 49 are adhered by the sealing resin 46c. Since the metal dots 44e are formed on the connecting surface of the semiconductor chip 44B sealed with the sealing resin 46c, it is possible to improve the adhesive force with the sealing resin 46c, and to prevent peeling of the insulating member of the semiconductor chip 44B.

### (Third Embodiment)

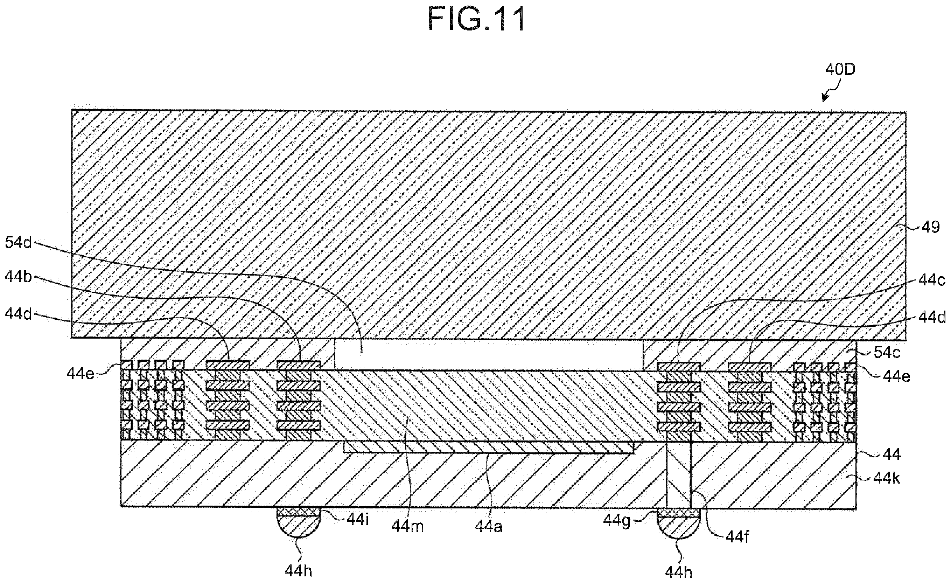

FIG. 11 is a partial cross-sectional view of an imaging unit according to a third embodiment of the present invention. FIG. 11 is a cross-sectional view of a connecting section between the protective glass 49 and the semiconductor chip 44 of the imaging unit according to the third embodiment of the present invention.

In an imaging unit 40D according to the third embodiment, an adhesive layer 54c which adheres the semiconductor chip 44 and the protective glass 49 has a hollow portion 54d on the light-receiving section 44a. The adhesive layer 54c is disposed on the peripheral circuit section 44b, the electrode pad 44c, the guard ring 44d and the metal dots 44e, except on the light-receiving section 44a, and the semiconductor chip 44 and the protective glass 49 are adhered with the adhesive layer 54c on the peripheral circuit section 44b, the electrode pad 44c, the guard ring 44d, and the metal dots 44e.

In the third embodiment, the adhesive layer 54c is disposed on the peripheral circuit section 44b, the electrode pad 44c, the guard ring 44d and the metal dots 44e, except on the light-receiving section 44a. Thus, it is possible to prevent entry of moisture from the adhesive surface between the semiconductor chip 44 and the protective glass 49. By providing a hollow portion 54d on the light-receiving section 44a, it is possible to prevent propagation of stress to the insulating layer 44m on the light-receiving section 44a with the adhesive layer 54c. Accordingly, it is possible to prevent peeling of the insulating member that constitutes the insulating layer 44m on the light-receiving section 44a.

### (Fourth Embodiment)

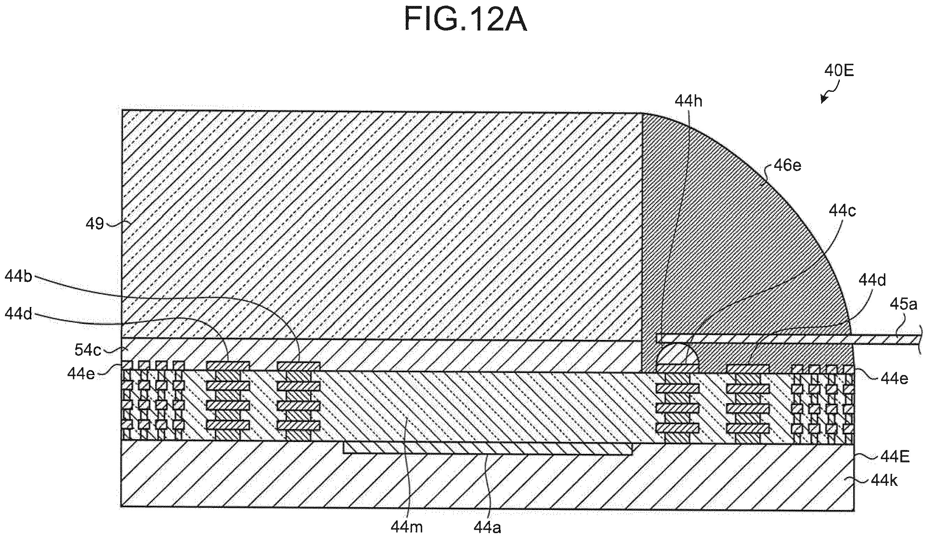

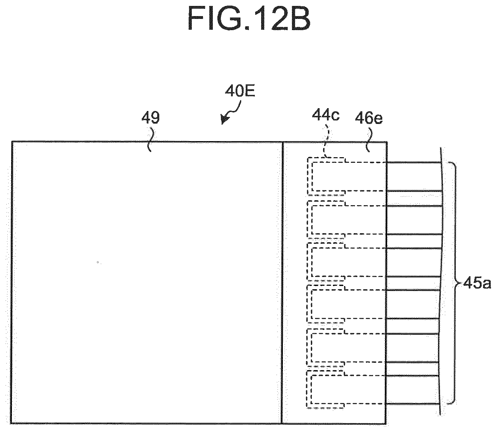

FIG. 12A is a partial cross-sectional view of an imaging unit according to a fourth embodiment of the present invention. FIG. 12B is a front view of the imaging unit according to the fourth embodiment of the present invention. FIG. 12A illustrates a cross-sectional view of a connecting section between the protective glass 49 and a semiconductor chip 44E of the imaging unit according to the fourth embodiment of the present invention.

In an imaging unit 40E according to the fourth embodiment, a through-electrode 44f, a back electrode 44g, and a dummy electrode 44i are not formed on the semiconductor substrate 44k, and an inner lead 45a extending from a FPC board via a bump 44h is connected to an electrode pad 44c of the connecting surface. Although it is not illustrated, the inner lead 45a is bent at the side surface of the semiconductor chip 44E and extends to the back side of the semiconductor chip 44E.

The planar dimension of the protective glass 49 orthogonal to the optical axis direction is formed to be smaller than that of the semiconductor chip 44E, and the protective glass 49 is adhered by the adhesive layer 54c to cover the guard ring 44d and the metal dot 44e of the three directions, except for the side of the light-receiving section 44a, the peripheral circuit section 44b, the electrode pad 44c, and the electrode pad 44c.

A sealing resin 46e is filled on the electrode pad 44c, the guard ring 44d, and the metal dot 44e of the semiconductor chip 44E which is not covered with the protective glass 49. In the imaging unit 40E according to the fourth embodiment of the present invention, the sealing resin 46e is filled on the connecting surface of the semiconductor chip 44E not covered with the protective glass 49, and the connecting surface of the semiconductor chip 44E and the side surface of the protective glass 49 are adhered by the sealing resin 46e. Since the metal dots 44e are formed on the connecting surface of the semiconductor chip 44E sealed with the sealing resin 46e, it is possible to improve the adhesive force with the sealing resin 46e and to prevent peeling of the insulating member of the semiconductor chip 44E.

### (Fifth Embodiment)

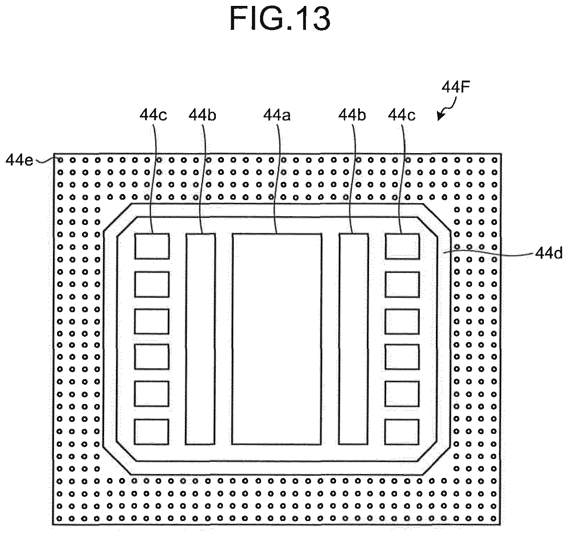

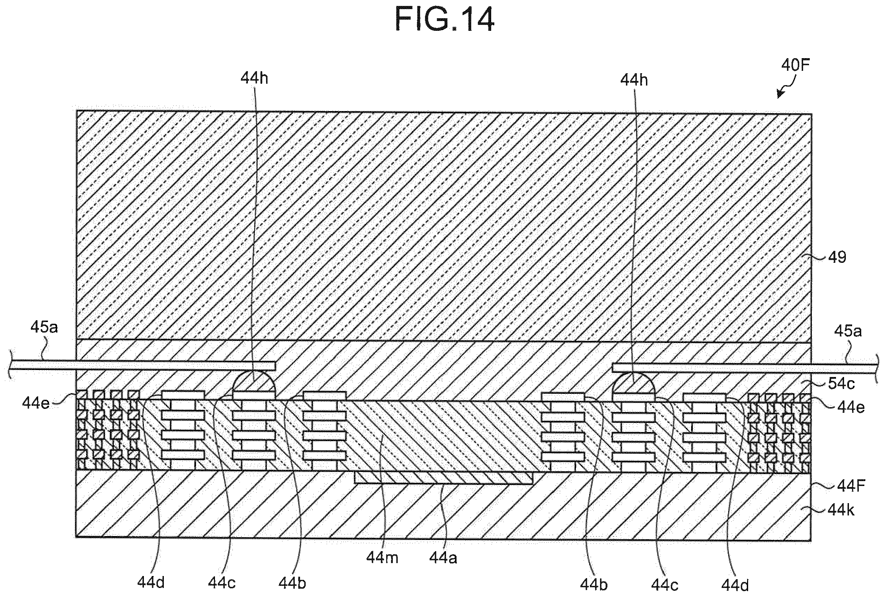

FIG. 13 is a plan view of a semiconductor chip used in the imaging unit according to the fifth embodiment of the present invention. FIG. 14 is a partial cross-sectional view of an imaging unit according to a fifth embodiment of the present invention, and illustrates a cross-sectional view of a connecting section between the protective glass and the semiconductor chip.

In an imaging unit 40F according to the fifth embodiment, as illustrated in FIG. 13, the peripheral circuit section 44b and the electrode pad 44c are formed on both sides with the light-receiving section 44a interposed therebetween, respectively. Inner leads 45a extending from the FPC board via the bump 44h are connected to the electrode pads 44c formed on both sides with the light-receiving section 44a interposed therebetween, respectively. The inner lead 45a is bent at the side surface of a semiconductor chip 44F and extends to the back side of the semiconductor chip 44F.

As illustrated in FIG. 14, the protective glass 49 is formed to have the same planar dimension orthogonal to the optical axis direction as the semiconductor chip 44F, and is adhered by the adhesive layer 54c cover the light-receiving section 44a, the peripheral circuit section 44b, the electrode pad 44c to which the inner lead 45a is connected, the guard ring 44d, and the plurality of metal dots 44e.

Even in the fifth embodiment, as in the first embodiment, even when a Low-k film or the like which is inferior in adhesion and mechanically fragile is used as the insulating member of the semiconductor chip 44F, since the plurality of metal dots 44e is disposed at the connecting end portions between the protective glass 49 prone to stress and the semiconductor chip 44F, peeling of the insulating member can be prevented. Further, since the metal dots 44e are formed on the outer circumferential portion of the semiconductor chip 44F, the semiconductor chip 44F can prevent chipping of the semiconductor substrate 44k in the process of dividing the semiconductor chip 44F.

### Reference Signs List

- 1: ENDOSCOPE SYSTEM
- 2: ENDOSCOPE DEVICE
- 3: UNIVERSAL CORD
- 3A: DISTAL END PORTION
- 3B: CURVED PORTION
- 3C: FLEXIBLE TUBE SECTION
- 4: OPERATING UNIT
- 4a: TREATMENT TOOL INSERTION OPENING
- 5: CONNECTOR UNIT
- 6: PROCESSOR
- 7: DISPLAY DEVICE
- 8: LIGHT SOURCE DEVICE
- 35: IMAGING DEVICE
- 40, 40A, 40B, 40C, 40D, 40E, 40F: IMAGING UNIT
- 41: DISTAL END PORTION MAIN BODY
- 41a: ADHESIVE
- 42: CLADDING TUBE
- 43: LENS UNIT
- 43a-1 to 43a-4: OBJECTIVE LENS
- 43b: LENS HOLDER
- 44, 44B, 44E, 44F: SEMICONDUCTOR CHIP
- 44a: LIGHT-RECEIVING SECTION
- 44b: PERIPHERAL CIRCUIT SECTION
- 44c: ELECTRODE PAD
- 44d: GUARD RING
- 44e: METAL DOT
- 44f: PENETRATION ELECTRODE
- 44g: BACK ELECTRODE
- 44h: BUMP
- 44i: DUMMY ELECTRODE
- 44k: SEMICONDUCTOR SUBSTRATE
- 44m: INSULATING LAYER
- 45: FLEXIBLE PRINTED CIRCUIT BOARD
- 45a: INNER LEAD
- 46: SEALING RESIN
- 47: ELECTRIC CABLE BUNDLE
- 48: SIGNAL CABLE
- 49: PROTECTIVE GLASS
- 50: HEAT SHRINKABLE TUBE
- 51: ADHESIVE RESIN
- 52: REINFORCING MEMBER
- 53: IMAGE SENSOR HOLDER
- 54a, 54b: SEALING RESIN
- 54c: ADHESIVE LAYER
- 55: to 57 ELECTRONIC COMPONENT
- 81: BENDING TUBE
- 82: BENDING WIRE

## Claims

1. An imaging unit comprising:
a semiconductor chip including an image sensor formed thereon; and
a protective glass adhered on the image sensor with an adhesive layer,
wherein the semiconductor chip comprises:
a light-receiving section configured to generate an image signal by performing photoelectric conversion of light;
a peripheral circuit section configured to receive the image signal from the light-receiving unit and transmit a driving signal to the light-receiving unit;
a guard ring surrounding the light-receiving section and the peripheral circuit section; and
a plurality of metal dots formed on an outer circumference of the guard ring, and
wherein the protective glass is adhered by the adhesive layer so as to cover the light-receiving section, the peripheral circuit section, the guard ring, and the metal dots.

2. The imaging unit according to claim 1, wherein the adhesive layer includes a hollow portion located above the light-receiving section, and
the protective glass is adhered by the adhesive layer so as to cover the peripheral circuit section, the guard ring, and the metal dots.

3. The imaging unit according to claim 1 or 2, wherein
the metal dots are further formed on a portion which is not covered with the protective glass, on a surface of the semiconductor chip on which the light-receiving section is formed, and
a sealing resin is filled on the metal dots formed in the portion not covered with the protective glass, and is adhered to a side surface of the protective glass.

4. The imaging unit according to claim 1 or 2, wherein a sealing resin is filled in a portion which is not in contact with the semiconductor chip, on a connecting surface between the protective glass and the semiconductor chip, and is adhered to a side surface of the semiconductor chip.

5. The imaging unit according to any one of claims 1 to 4, wherein the guard ring and the metal dots are formed of dummy vias and dummy pads formed on a plurality of insulating members laminated on a semiconductor substrate on which the light-receiving section is formed, respectively, and
the plurality of insulating members are Low-k films.

6. An endoscope device comprising an insertion section including the imaging unit according to any one of claims 1 to 5 provided at a distal end.
